# EUROPÄISCHE PATENTSCHRIFT

(11) **EP 0 480 275 B1**
(45) Veröffentlichungstag und Bekanntmachung des Hinweises auf die Patenterteilung: **11.01.1995**
(21) Anmeldenummer: 91116647.8
(22) Anmeldetag: 30.09.1991
(51) Int. Cl.: B23K 26/02, B23K 26/00

(54) **Kraftgesteuerter Kontaktapplikator für Laserstrahlung**
Power controlled contact applicator for laser irridiation
Appui de contact avec force contrôlée pour rayonnement du laser

(30) Priorität: 12.10.1990 DE 4032860
(43) Veröffentlichungstag der Anmeldung: 15.04.1992
(73) Patentinhaber: Firma Carl Zeiss, D-73446 Oberkochen (DE); CARL ZEISS-STIFTUNG HANDELND ALS CARL ZEISS, D-89518 Heidenheim (Brenz) (DE)
(72) Erfinder: Müller, Gerhard, Prof. Dr., W-1000 Berlin 38 (DE); Müller-Stolzenburg, Norbert, Dr., W-1000 Berlin 45 (DE)

(56) Entgegenhaltungen:
- EP-A- 0 307 544
- PATENT ABSTRACTS OF JAPAN vol. 8, no. 80 (M-289)(1517) 12. April 1984 & JP-A-58 224 091 (TOSHIBA KIKAI K.K.) 26 Dezember 1983

## Beschreibung

Die Erfindung betrifft einen Laserapplikator der im Oberbegriff des Anspruchs 1 angegebenen Art.

Es ist bekannt, Laserstrahlung im Bereich zwischen 300 nm und 3 µm durch optische Lichtwellenleiter zu übertragen und damit eine Materialbearbeitung im weitesten Sinne durchzuführen. Hierunter sollen auch Anwendungen in medizinischen Bereich verstanden werden.

Es ist ebenfalls bekannt, daß bei heterogenen Materialien infolge von Vielfachstreuung an Texturgrenzen der Wirkenteil der applizierten Laserleistung im eigentlichen Zielvolumen nur schwer kontrollierbar ist, so daß das sich einstellende Arbeitsergebnis für eine fest eingestellte konstante Laserleistung nur schwer abschätzbar ist.

Der Erfindung liegt die Aufgabe zugrunde, einen Laserapplikator der eingangs genannten Gattung derart zu gestalten, daß die Laserstrahlung noch bei der Anwendung selbst mit kontrollierten Parametern appliziert werden kann, ohne daß zusätzliche Einstellvorrichtungen oder Hilfsapparaturen betätigt werden müßten.

Diese Aufgabe wird mit den Merkmalen des Anspruchs 1 gelöst.

Der Erfindung liegt die Erkenntnis zugrunde, daß bei verschiedenen Anwendungen in Industrie und Medizin die Notwendigkeit besteht, Laserstrahlung unter vorbestimmten Kraftbedingungen aufzubringen bzw. dafür zu sorgen, daß erst bei Erreichen einer bestimmten Andruckkraft des optischen Lichtwellenleiters auf dem zu bearbeitenden Material die Laserstrahlung auszulösen. Vielfach muß die Laserstrahlung auch über einen bestimmten Zeitraum einwirken. Dies ist bei den verschiedensten Anwendungen in der Mikroelektronik und Mikromaterialbearbeitung der Fall, wie beispielsweise bei mechanisch verformbaren Verbundmaterialien.

Hierzu sind generell zwei Wege gangbar:
1. Es ist ein über einen Hubbereich von einer Quelle für eine möglichst konstante Kraft angetriebenes, vorzugsweise federnd gelagertes, Element vorgesehen, welches bei der Laserbestrahlung an der Materialoberfläche anliegt.
2. Es ist eine Meßvorrichtung für die Andruckkraft vorgesehen, welche durch ein akustisches Signal oder Einschaltung der Laserquelle der Bediengungsperson zu erkennen gibt, daß die ausgeübte Andruckkraft sich innerhalb eines vorgebenen Bereichs befindet.

Diese beiden Wege sind in der Weise miteinander kombinierbar, daß das Meßelement für die Andruckkraft nach 2. ein federnd gelagertes Element - und nicht einen im wesentlichen starr gehaltenen Kraftaufnehmer - bildet.

Besonders vorteilhaft ist die erfindungsgemäße Lösung auch in der Medizin einsetzbar - wo beispielsweise zur Verringerung der Streuung und besseren Haftung - äußere Gewebeschichten zunächst mechanisch mit tieferen Gewebeschichten in innigen Kontakt gebracht werden müssen, bevor über die ausgelöste Laserstrahlung eine Koagulationsnekrose zur Verbindung der Gewebeschichtung gesetzt wird.

Günstig bei der Erfindung ist auch, daß mittels der kontrollierten Krafteineinleitung über das Strahlführungssystem des Lasergerätes Zielstrukturen, insbesondere heterogene Verbundmaterialien, wie sie in verschiedenen Anwendungsfeldern der Mikroelektronik und Mikrostrukturtechnik - und inbesondere in der Medizin - vorkommen, in wesentlich genauerer Dosierung mit Laserenergie beaufschlagt werden können.

Dabei ist bei der Anwendung im medizinischen Bereich zu berücksichtigen, daß Gewebestrukturen bei den dort zur Anwendung gekommenen Strahlfülhrungssystemen in aller Regel - aber nicht ausschließlich - einen optischen Lichtwellenleiter verwenden und infolgedessen durch den mechanischen Kontakt der Gewebeoberfläche bei zu großem Andruck Verletzungsgefahr besteht. Auf der anderen Seite ist es aber erwünscht, mit einer bestimmten maximal möglichen Kraft Gewebeschichten soweit zu komprimieren, daß durch die zelluläre Textur bedingte Vielfachstreuung auf ein Minimum gesenkt wird und damit näherungsweise die volle übertragende Laserleistung am Zielort zur Verfügung steht.

Um einerseits sicherzustellen, daß die Einleitung der Laserstrahlung optimal erfolgt, andererseits aber bei Überschreitung des zulässigen Andrucks dem Bediener den Gefahrenzustand zu signalisieren, sind bei einer günstigen Weiterbildung der Erfindung zwei Schaltpunkte vorgesehen, bei denen - mit Zunahme der aufgebrachten Andruckkraft - der Laser zunächst beim ersten ersten Schaltpunkt an und dann beim zweiten wieder ausgeschaltet wird. Auf diese Weise können die bevorzugten oder notwendigen Arbeitsbedingungen ohne besondere zusätzlich Aufmerksamkeit auf Seiten des Bedieners eingehalten werden und es sind insbesondere auch nicht vom Arbeitsbereich entfernt gelegene Bedienungselemente zu betätigen so daß jederzeit die volle Konzentration ausschließlich dem Arbeitsfeld zugewandt werden kann.

Der Druck auf die Arbeitsfläche wird von einem am Handstück befestigten Fühlelement abgefragt, das vorzugsweise auf in Richtung der Faser wirkende Druckkräfte anspricht. Es ist bevorzugt relativ zu dem Handstück federnd verschieblich gelagert. Dabei kann das Fühlelement sowohl durch das Faserende als auch durch ein die Faser umgebendes Hüllrohr gebildet werden. Im erstgenannten Fall ist das Ende mechanisch gefaßt und in einem übergeschobenen äußerem mechanischen Hüllrohr verschieblich gelagert.

Zur kontrollierten Krafteinleitung ist dann entweder eine Feder an der mechanischen Fassung der Faser oder am Hüllrohr mechanisch angelenkt und je nach bevorzugter Lösung wird dann die aus dem Hüllrohr herausragende Faser entgegen der Federkraft gegen einen voreinstellbaren Anschlag in das Hüllrohr zurückgeschoben bzw. das in Bezug auf das dem Faserende hervorstehende Hüllrohr gegenüber der fixierten Faser verschoben, bis ebenfalls wieder durch einen voreinstellbaren Anschlag die gewünschte Kraft auf das Gewebe ausgeübt wird.

In einer anderen bevorzugten Ausführungsform wird über den voreinstellbaren Anschlag des Federweges mittelbar ein elektrischer Kontakt betätigt, der dann die Laserstrahlung auslöst.

In Weiterführung des Erfindungsgedankens können auf dem mechanisch ausgelenkten Federweg auch zwei Kontakte vorgesehen werden, die dann über die Federkraft einen unteren und oberen Grenzwert für die mechanisch aufzubringende Kraft zur Komprimierung der Zielstruktur darstellen, wobei dann der obere Grenzwert zum Abschalten des Lasers und Auslösen eines Alarmsignales genutzt werden kann.

In einer bevorzugten Ausführungsform für die sogenannte Zyklokoagulation am Auge ist eine am distalen Ende hemisphärisch verrundete Faser mit einer mechanisch festen Hülse mit Anschlag versehen. Vorzugsweise besteht diese Hülle aus Metall mit aufgefalztem Ring und kann in dem inneren Führungsteil des Handstückes mit einer Schnellverschraubung mit der Federanlenkung verbunden sein. Im Handstück selber werden die elektrischen Kontakte der Grenzwertmelder des Federauslenkweges, die entsprechend der gewünschten Andruckkraft eingestellt werden können, in Serie mit einem auslösenden Fußschalter mit der Steuerung des Lasergeräts verbunden.

Mechanische kraftgesteuerte Elemente an strahlungsführenden Hohlleitern oder Mehrspiegelgelenkarmen sind ebenfalls in günstiger Weise zur Anwendung mit der Erfindung geeignet.

Der insoweit betriebene kraftgesteuerte Applikator weist durch den konstruktionsbedingten Federweg eine mechanische Rückstellkraft auf, die gegebenenfalls bei verschiedenen Anwendungen, insbesondere im Falle der wünschenswerten Perforation von Gewebeschichten, störend sein kann. Dieses Problem, das sich bei Einwirken einer bestimmten Kraft der Applikator um einen bestimmten Weg verkürzt, auf der anderen Seite das Gewebe mechanisch deformiert wird, kann im Fall der gezielten und gewünschten Perforation dazu führen, daß bei plötzlichem Ausbleiben der Gegenkraft des Gewebes die druckapplizierende Faser vorschnellt. Um dieses zu verhindern, wird in Weiterführung des Erfindungsgedankens anstelle der eingangs vorgesehenen Spiralfeder lediglich eine relativ steife membran- oder blattfederartige Halterung des jeweils beweglichen Teils des Kraftapplikators, sei es nun der Faser oder des übergreifenden Hüllrohres, vorgesehen. Die elastischen Auslenkungen dieser membran- oder blattfederartigen Halterung können dann, falls gewünscht, sogar weniger als einen Zehntel Millimeter betragen und über einen Dehnungsmeßstreifen gemessen werden. Dieses elektrische Signal des Dehnungsstreifens wird dann einer Auswerterelektronik zugeführt, mit der wiederum kraftproportionale Ein- und Ausschaltgrenzwerte für den Laser einstellbar sind.

Durch den Nachweis der aufgebrachten Kraft über Dehnungsmeßstreifen besteht eine sehr präzise und feinfühlige Möglichkeit, den Toleranzbereich für die Kraftleitung gleichzeitig als Auslösekriterium für die Laserstrahlung zu nutzen und somit hochgenau Laserstrahlung und Kontakt bei exakt vorgegebener Krafteinleitung anzuwenden. Mit dieser erfindungsgemäßen Lösung der über Dehnungsmeßstreifen oder entsprechend Kraftmeßdosen bzw. kapazitive oder induktive Wegaufnehmer einstellbaren Ein- und Ausschaltkriterien der Laserstrahlung ergeben sich auch weitere Anwendungsmöglichkeiten des erfindungsgemäßen Grundgedankens des kraftgesteuerten Laserapplikators.

Insbesondere ist es damit auch möglich, Laserkatheter in der Angioplastie mit kontrollierter Krafteinleitung voranzuschieben. Genauso gut können damit aber auch laserstrahlungsführende optische Fasern, mit denen nicht nur gebohrt oder koaguliert werden soll und die in axialer Richtung mit Kraft beaufschlagt werden bei Anbringung der entsprechenden Dehnungsmeßstreifen zur Messung der Querkraft, dann auch zum Schneiden von Gewebe bei kontrollierter Krafteinleitung verwendet werden.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1a ein erstes Ausführungsbeispiel des erfindungsgemäßen Kontaktapplikators im Schnitt,
Figur 1b ein zweites Ausführungsbeispiel des erfindungsgemäßen Kontaktapplikators im Schnitt,
Figur 1c eine Detaildarstellung einer weiteren Variante des erfindungsgemäßen Kontaktapplikators,
Figur 2a ein Blockschaltbild einer ersten Ausführung für den erfindungsgemäßen Kontaktapplikator, sowie
Figur 2b eine zweite Ausführung eines Steuerteils.

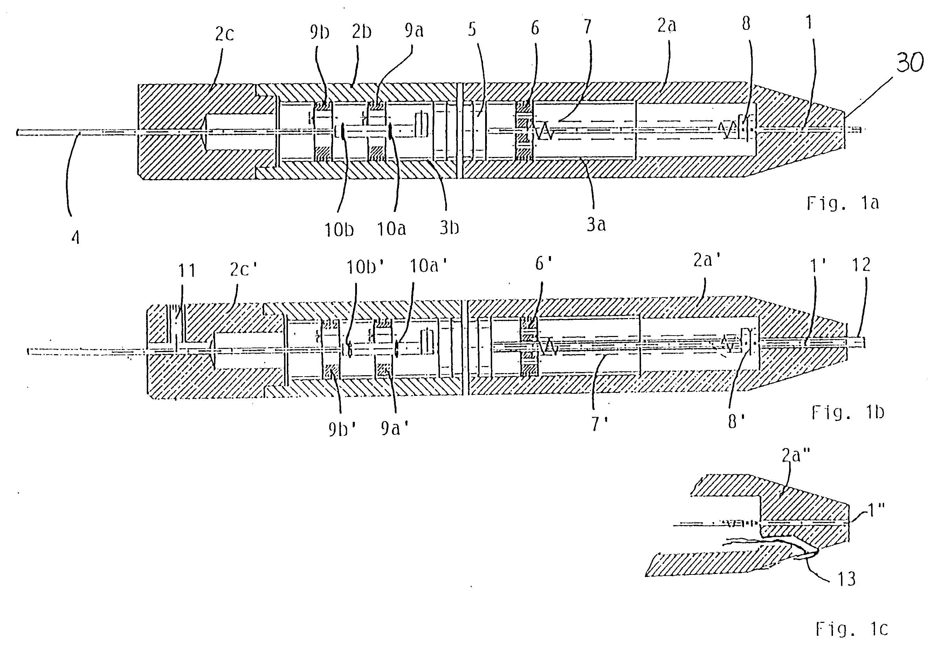

Bei dem in Figur 1a dargestellten Kontaktapplikator ist ein Glasfaserleiter 1 in einem dreiteiligen Handstück, bestehend aus den Segmenten 2a bis 2c, verschieblich gelagert. Das Handstück 2a bis 2c hat etwa die Form eines Schreibgerätes, wobei der Glasfaserleiter die Position der Schreibmine hat. Die Segmente 2a und 2b sind mit einer durchgehenden Bohrung versehen, wobei erweiterte Bereiche 3a und 3b mindestens teilweise mit einem Innengewinde versehen sind. Das Abschlußsegment 2c verschließt das Segment 2b und weist seinerseits einen Durchlass für eine Glasfaserzuleitung auf. Die Segmente 2a und 2b sind mit einem Kupplungsstück verbunden, welches wiederum eine durchgehende Bohrung für den Durchlass der Glasfaser aufweist. Das Handstück hat insgesammt etwa eine Länge von 120 mm bei einem Durchmesser von 16 mm. Der Glasfaserleiter 1 ragt um ca. 3mm aus dem zugespitzten Ende des Segmentes 2a heraus. Im Innengewinde der Ausnehmung 3a ist ein verstellbares Widerlager 6 vorgesehen, an dem sich eine den Glasfaserleiter 1 im Inneren des Segmentes 2a umgebende Schraubenfeder 7 abstützt, welche mit ihrem anderen Ende an einer Stellring 8 anliegt, der mit dem Glasfaserleiter 1 fest verbunden ist und einen Anschlag gegen ein weiteres Ausfahren des Glasfaserleiters 1 aus dem Segment 2a bildet.

Bei Benutzung des Handstücks ist somit eine Mindestkraft erforderlich, um bei Andruck der Spitze des Faserleiters 1 auf das zu behandelnde Objekt den einen Anschlag bildenden Stellring aus seiner Anschlagposition abzuheben. Bis zum völligen Eindrücken des Faserleiters in eine Position, in der seine Frontfläche bündig mit der Frontfläche des Segmentes 2a des Handstücks ist, ändert sich die aufzubringende Kompressionskraft wegen der Länge der Schraubenfeder 7 nur geringfügig. Wenn der Operateur also einen federnden Widerstand spürt, ist sichergestellt, daß er mit einer innerhalb des durch die vorgenommenen Grenzwerte beschränkten Toleranzbereichs einen Druck auf das zu behandelnde Gewebe ausübt.

Um die Kontrollmöglichkeit weiter zu verbessern, sind Schaltmittel vorgesehen, welche in Abhängigkeit von der Position des Faserleiters 1 Signale abgeben, um die Laserquelle einzuschalten sobald der auf die Oberfläche auszuübende Mindestdruck erreicht ist und wieder auszuschalten, sobald dieser Druck überschritten wird. Diese beiden Grenzwerte sind identisch mit der nahezu voll ausgefahrenen Position des Lichtleiters 1 und der nahezu voll eingefahrenen Position. Im Segment 2b des Handstücks sind dazu in ihrer axialen Position mittels des dort vorhandenen Innengewindes feineinstellbare Schaltelemente 9a und 9b vorgesehen, welche durch mit dem Faserleiter 4 verbundene Aktivierungselemente 10a und 10b betätigt werden. Sobald durch ein Fahren des Faserleiters um einen Minimalbetrag das Element 10a benachbart zum Schaltlement 9a ist wird ein erstes Signal abgegeben, welches die Laserquelle - wie weiter unten beschrieben - einschaltet bzw. ein entsprechendes Aufmerksamkeitssignal auslöst, welches der Bedienungsperson das Einhalten eines vorgegebenen Bereichs der Andruckkraft signalisiert. Nähert sich das Element 10b dem Schaltelement 9b, so wird durch ein weiteres entsprechendes Signal die Laserquelle wieder ausgeschaltet. Auf diese Weise ist der Benutzer intuitiv in der Lage die vorgegebenen Kraftgrenzwerte einzuhalten.

Das in Figur 1b dargestellte Ausführungsbeispiel ist entsprechend aufgebaut, so daß übereinstimmend die Elemente mit entsprechenden Bezugszeichen versehen werden konnten, denen jeweils ein (') hinzugefügt wurde. Bei dem in Figur 1b dargestellten Ausführungsbeispiel ist der Faserleiter 1' jedoch in einer Position, mit der seine Frontfläche bündig mit der jenigen des vorderen Segments 2a' des Handstücks abschließt arretiert. Der Faserleiter ist damit durchgehend bis zum hinteren Ende des Handstücks (Segment 2c') ausgebildet und wird in diesem Segment mittels einer Stellschraube 11 festgehalten. Zum Abfragen des Anpressdrucks ist zwischen Faserleiter und Handstück ein relativ zu diesem verschiebliches Röhrchen 12 vorgesehen, welches nun seinerseits im entlasteten Zustand aus der Frontfläche des Handstücks um ca. 3 mm herausragt. Dieses Röhrchen ist wiederum mit einem Stellring 8' mit einer Schraubenfeder 7' verbunden, welche sich an einem Element 6' abstützt. Anstelle des Faserleiters übernimmt bei der Ausführung gemäß Figur 1b das den nunmehr festen Faserleiter 1' umgebende Röhrchen die Funktion der Kraftaufbringung und aktiviert auch die Betätigungselemente 10a' und 10b', welche bei Verschiebung relativ zu den Schaltelementen 9a' und 9b' in entsprechender Weise ein Ausschalten der Laserquelle bewirken.

Wenn auf die durch den Hub des Lichtleiters (Figur 1a) oder Röhrchen (Figur 1b) ausgelöste mechanische Rückmeldung für den Operateur verzichtet werden soll, so können anstelle der durch bewegliche Kontakte betätigten Schaltelemente und anstelle der Schraubenfeder als Element zur Kraftaufbringung auch Drucksensoren verwendet werden, welche mit nur geringem oder auch ohne Hub arbeiten und dabei ein elektrisches Signal abgeben, welches die Laserquelle in einem vorgegebenen Kraftandruckbereich zunächst ein- und dann wieder ausschaltet. Als derartige Elemente kommen Dehnungsmeßstreifen, Piezoelemente und dergleichen in Betracht. Sie können im Handstück anstelle der Elemente 9/10 angeordnet sein und geben das entsprechende Ausgangssignal ab, sobald zwischen diesen Elementen eine entsprechende Betätigungskraft abnehmbar ist.

Bei dem in Figur 1c dargestellten Ausführungsbeispiel ist ein Lichtleiter 1'' im vorderen Segment 2a'' fest angebracht. Ein Druckaufnehmer 13, der mit kurzem bzw. ohne Hub arbeitet ist quer zur Richtung des Faserleiters 1'' angeordnet. Auf diese Weise sind mit dem Handstück schneidende Bewegungen möglich, wobei der Druckaufnehmer 13 die seitliche Anlagekraft erfasst und in entsprechender Weise ein- bzw. ausschaltet.

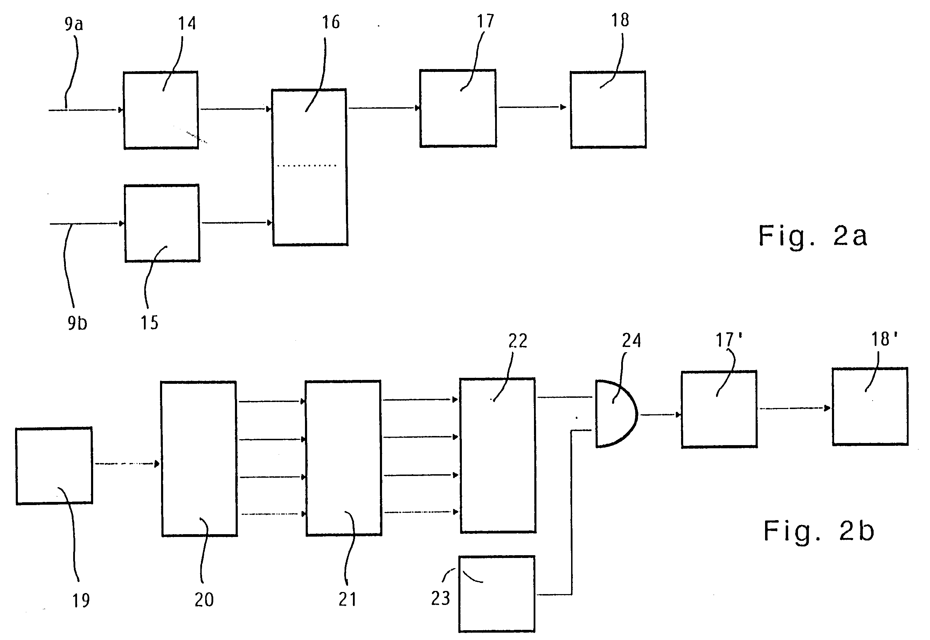

In Figur 2a ist ein elektrischer Steuerteil wiedergegeben, welcher die entsprechenden Schaltvorgänge ausführt. Hierbei werden die Ausgangssignale der Schaltelemente 9a und 9b Meßwandlern 14 und 15 zugeführt, welche ihrerseits ein digitales Ausgangssignal abgeben, welches ein Flip-Flop 16 setzt bzw. zurücksetzt. Das logische Ausgangssignal Q des Flip-Flops 16 dient zur Ansteuerung der Energiequelle 17 für einen Laser 18. (Es ist ersichtlich, daß mit dem Setzen bzw. Zurücksetzen des Flip-Flops 16 bei Abgabe des Ein- bzw. Ausschaltsignals die entsprechende Aktivierung des Lasers erfolgt.)

Bei dem in Figur 2b dargestellten Blockschaltbild ist ein Meßwandler 19 vorgesehen, welcher ein analoges, der Position des Lichtleiters 1, oder des Röhrchens 12 bzw. ein anderes zur anliegenden Kraft proportionales Signal abgibt. Dieses Signal wird einem Digital-Analog-Wandler 20 zugeführt, der an verschieden Signalausgängen parallel eine Digitalinformation abgiebt, welche einen Dekoder 21 ansteuert. Dieser Dekoder ist in der Lage auch zeitliche Änderungen des Eingangssignals auszuwerten, so daß auf vorgegebene Signalein- bzw. Auschaltzeiten hin oder das Überschreitcn bestimmter Signalpegel für vorgegebene Zeiten eine dementsprechenden zeitlichen Verlauf des Eingangssignals zugeordnetes digitales Ausgangssignal abgibt. Dieses Ausgangssignal adressiert in einem Speicher 22 eine vorprogrammierte Schaltfunktion, welche ebenfalls zeitabhängig sein kann. Auf diese Weise lassen sich - nach entsprechender Programmierung des Dekoders bzw. des Speichers - beliebige zeitabhängige Verläufe der Lasersteuerung beliebigen zeitabhängigen Verläufen der aufgebrachten Kraft zuordnen. Damit kann die beispielsweise durch kurz aufgebrachte Kraftimpulse oder Impulsfolgen oder kurzfristiges Nachlassen des Kraftdruckes in beliebiger Weise heraufbzw. heruntergeregelt werden oder aber es können zeitabhängig vorprogrammierte Intensitätsverläufe abgerufen werden. Auf diese Weise ist einhändiges Arbeiten in der Weise möglich, daß sich der Operateur vollständig auf den Behandlungsbereich konzentrieren kann. Als zusätzliches Sicherheitselement kann ein Fußschalter vorgesehen sein, welcher mittels eines UND-Gatters das Ausgangssignal des Speichers 22 in der Weise beeinflussen kann, daß die Energiequelle 17' des Lasers 18' nur dann aktivierbar ist, wenn zusätzlich der Fußschalter betätigt ist.

## Patentansprüche

1. Kontaktapplikator für Laserstrahlung mit einem Strahlführungssystem,
**dadurch gekennzeichnet**,
daß an der Strahlaustrittsseite des Kontaktapplikators vorgesehen ist:
ein aus der Oberfläche (30) hervortretendes in einem vorgegebenen Kraftbereich federnd abgestütztes Fühlelement (1, 12),
oder
ein druckempfindliches Betätigungselement (13), welches bei Anlage der Strahlaustrittsseite des Strahlführungsystems an ein Objekt aktiviert wird,
wobei der Ausgang des Betätigungselements (13) oder des Fühlelementes (1, 12) mit einem Steuerteil zur Beeinflussung der Intensität der Laserstrahlung verbunden ist.

2. Kontaktapplikator nach Anspruch 1, **dadurch gekenn** **zeichnet**, daß die Beeinflussung der Intensität der Laserstrahlung oder die Erzeugung eines akustischen bzw. eines sonstigen Aufmerksamkeitssignals in Abhängigkeit von der Andruckkraft erfolgt.

3. Kontaktapplikator nach einem der vorangehenden Ansprüche, **dadurch gekennzeichnet**, daß in Abhängigkeit vom Verschiebeweg des Fühlelements (1, 12) aktivierte Schalmittel (9a, 9b) vorgesehen sind, deren Ausgangsignal ein Eingangssignal für die Steuermittel zur Beeinflussung der Intensität der Laserstrahlung bildet.

4. Kontaktapplikator nach einem der vorangehenden Ansprüche, **dadurch gekennzeichnet**, daß die Schaltmittel (9a, 9b) aus einem Potentiometer bestehen, dessen Schleifer mit dem Fühlelement (1, 12) mechanisch verbunden ist.

5. Kontaktapplikator nach einem der vorangehenden Ansprüche, **dadurch gekennzeichnet**, daß die Schaltmittel (9a, 9b) aus einer Kraftmeßdose bestehen, dessen Meßeingang mit dem Fühlelement (1, 12) mechanisch verbunden ist.

6. Kontaktapplikator nach einem der vorangehenden Ansprüche, **dadurch gekennzeichnet**, daß die Schaltmittel (9a, 9b) aus einem kapazitiven Kraftaufnehmer bestehen, dessen Schleifer mit dem Fühlelement (1, 12) mechanisch verbunden ist.

7. Kontaktapplikator nach einem der vorangehenden Ansprüche, **dadurch gekennzeichnet**, daß die Schaltmittel (9a, 9b) aus einem Dehnungsmeßstreifen bestehen, der mit dem Fühlelement (1, 12) mechanisch verbunden ist.

8. Kontaktapplikator nach einem der vorangehenden Ansprüche, **dadurch gekennzeichnet**, daß die Schaltmittel (9a, 9b) aus einem induktiven Wegaufnehmer bestehen, dem mit dem Fühlelement (1, 12) mechanisch verbunden ist.

9. Kontaktapplikator nach einem der vorangehenden Ansprüche**, dadurch gekennzeichnet**, daß das optische Strahlführungssystem aus einem Lichtwellenleiter oder einem Spiegelgelenksystem besteht.

10. Kontaktapplikator nach einem der vorangehenden Ansprüche, **dadurch gekennzeichnet**, daß das Fühlelement (1, 12) ein Rohr (12) bildet, welches coaxial zum Strahlführungssystem gelagert ist.

11. Kontaktapplikator nach einem der vorangehenden Ansprüche, **dadurch gekennzeichnet**, daß die Laserstrahlung nach Überschreitung einer ersten einstellbaren Grenzkraft ein- und/oder nach Überschreitung einer zweiten einstellbaren Grenzkraft ausgeschaltet wird.

12. Kontaktapplikator nach einem der vorangehenden Ansprüche, **dadurch gekennzeichnet**, daß die Laserstrahlung nach einem von mehreren vorgegebenen Programmen zeitabhängig in ihrer Intensität gesteuert wird, wobei die Programmauswahl mittels eines Decoders durch Diskrimation der Über- bzw. Unterschreitung von vorgegebenen Grenzkräften bei einem oder mehreren Fühlelementen (1, 12) innerhalb eines Zeitrasters erfolgt.

13. Kontaktapplikator nach einem der vorangehenden Anspruch 1, **dadurch gekennzeichnet**, daß das Fühlelement (1, 12) durch die Faser selbst gebildet ist, welche in Bezug auf ein Handstück verschieblich gelagert ist.

14. Kontaktapplikator nach Anspruch 1, **dadurch gekenn zeichnet**, daß die Strahlungsrichtung eine quer zur Längsachse eines Handstücks gerichtete Richtungskomponente aufweist.

15. Kontaktapplikator nach Anspruch 1, **dadurch gekenn zeichnet**, daß die Auslösung der Laser-Strahlung mittels logischer UND-Verküpfung von einem weiteren Betätigungselement, insbesondere einem Fußschalter, abhängig ist.

## Claims

1. Contact applicator for laser radiation with a radiation guide system,
characterized by the fact,
that said contact applicator has a radiation outlet surface with:
a sensing element (1, 12) which extends outwardly beyond the surface (30) and which is resiliently biased within a pregiven force range,
or
a pressure sensitive actuating element (13) which is activated when the radiation outlet surface of the radiation guide system is in contact with an object,
whereas the output of the actuating element (13) or the sensing element (1, 12) is connected with a control system to influence the intensity of the laser radiation.

2. Contact applicator according to claim 1, characterized by the fact, that the intensity of the laser radiation or the generation of an acoustic alarm signal or another alarm signal is dependent of the contact force.

3. Contact applicator according to one of the preceding claims, characterized by the fact, that there are switching means (9a, 9b) which can be activated in dependency of the displacement of the sensing element (1, 12) and which are generating an output signal for the control means to influence the intensity of the laser radiation.

4. Contact applicator according to one of the preceding claims, characterized by the fact, that the switching means (9a, 9b) being a potentiometer having a slip ring which is mechanically connected with the sensing element (1, 12).

5. Contact applicator according to one of the preceding claims, characterized by the fact, that the switching means (9a, 9b) being a pressure gauge having a measuring input which is mechanically connected with the sensing element (1, 12).

6. Contact applicator according to one of the preceding claims, characterized by the fact, that the switching means (9a, 9b) being a capacitive force sensor having a slip ring which is mechanically connected with the sensing element (1, 12).

7. Contact applicator according to one of the preceding claims, characterized by the fact, that the switching means (9a, 9b) being a wire strain gauge which is mechanically connected with the sensing element (1, 12).

8. Contact applicator according to one of the preceding claims, characterized by the fact, that the switching means (9a, 9b) being an inductive displacement sensor which is mechanically connected with the sensing element (1, 12).

9. Contact applicator according to one of the preceding claims, characterized by the fact, that the optical radiation guide system being a light wave conductor or an articulated arm mirror system.

10. Contact applicator according to one of the preceding claims, characterized by the fact, that the sensing element (1, 12) being a tube (12) which is mounted coaxially in relation to the radiation guide system.

11. Contact applicator according to one of the preceding claims, characterized by the fact, that the laser radiation is switched on after reaching an adjustable first force limit value and/or the laser radiation is switched off after reaching an adjustable second force limit value.

12. Contact applicator according to one of the preceding claims, characterized by the fact, that the laser radiation is controlled with a time dependent intensity according to one of several pregiven programs, whereas the selection of the programs is achieved by a decoder which discriminates the signals of one or more sensing elements (1, 12) which are higher or lower than certain pregiven force limit values within a certain time raster.

13. Contact applicator according to one of the preceding claims, characterized by the fact, that the sensing element (1, 13) being a light wave conductor, which is mounted displaceably within a hand piece.

14. Contact applicator according to claim 1, characterized by the fact, that the laser radiation having a radiation component which is arranged crosswise in relation to the longitudinal axis of the hand piece.

15. Contact applicator according to claim 1, characterized by the fact, that the activation of the laser radiation via a logical AND gate is dependent of a further activating element, especially a foot switch.

## Revendications

1. Tube guide de contact pour l'application d'un rayonnement laser, doté d'un système de guidage du faisceau,
**caractérisé en ce que**
la face de sortie du faisceau du tube guide de contact est munie
d'un capteur (1, 12) qui s'élève au-dessus de la surface (30) et qui est monté sur ressort de manière à céder dans une plage de forces d'appui prédéfinies
ou
d'un actionneur sensible à la pression (13) qui est activé lorsqu'on applique la face de sortie du faisceau du système de guidage sur un objet, la sortie dudit actionneur (13) ou dudit capteur (1, 12) étant reliée à une unité de commande servant à influer sur l'intensité du rayonnement laser.

2. Tube guide de contact selon la revendication 1, **caractérisé en ce que** l'intensité du rayonnement laser est dosée ou un signal d'avertissement acoustique ou autre généré en fonction de la force de pression produite.

3. Tube guide de contact selon l'une quelconque des revendications précédentes, **caractérisé en ce que** des éléments interrupteurs (9a, 9b) sont prévus qui sont activés en fonction de la distance de translation du capteur (1, 12) et dont le signal émis forme le signal d'entrée des moyens de commande influant sur l'intensité du rayonnement laser.

4. Tube guide de contact selon l'une quelconque des revendications précédentes, **caractérisé en ce que** les éléments interrupteurs (9a, 9b) sont constitués par un potentiomètre dont le curseur est solidaire du capteur (1, 12).

5. Tube guide de contact selon l'une quelconque des revendications précédentes, **caractérisé en ce que** les éléments interrupteurs (9a, 9b) sont constitués par une boîte dynamométrique dont l'entrée de mesure est solidaire du capteur (1, 12).

6. Tube guide de contact selon l'une quelconque des revendications précédentes, **caractérisé en ce que** les éléments interrupteurs (9a, 9b) sont constitués par un transducteur de force capacitif dont le curseur est solidaire du capteur (1, 12).

7. Tube guide de contact selon l'une quelconque des revendications précédentes, **caractérisé en ce que** les éléments interrupteurs (9a, 9b) sont constitués par une jauge extensométrique qui est solidaire du capteur (1, 12).

8. Tube guide de contact selon l'une quelconque des revendications précédentes, **caractérisé en ce que** les éléments interrupteurs (9a, 9b) sont constitués par un capteur de déplacement inductif qui est solidaire du capteur (1, 12).

9. Tube guide de contact selon l'une quelconque des revendications précédentes, **caractérisé en ce que** le système de guidage optique du faisceau laser est constitué par un guide d'ondes lumineuses ou d'un système articulé à miroirs.

10. Tube guide de contact selon l'une quelconque des revendications précédentes, **caractérisé en ce que** le capteur (1, 12) forme un tube qui est disposé coaxialement par rapport au système de guidage du faisceau.

11. Tube guide de contact selon l'une quelconque des revendications précédentes, **caractérisé en ce que** le rayonnement laser est coupé en cas de dépassement d'une première force limite réglable et/ou en cas de sousdépassement d'une seconde force limite réglable.

12. Tube guide de contact selon l'une quelconque des revendications précédentes, **caractérisé en ce que** l'intensité du rayonnement laser est commandée en fonction du temps par l'un de plusieurs programmes, le programme étant sélectionné par l'intermédiaire d'un décodeur, par discrimination du dépassement ou du sous-dépassement de forces limites prédéfinies sur un ou plusieurs capteurs (1, 12) dans un système à tranches de temps.

13. Tube guide de contact selon l'une quelconque des revendications précédentes, **caractérisé en ce que** le capteur (1, 12) est formé par la fibre optique elle-même qui est disposée de manière à pouvoir être déplacée par rapport à une pièce à main.

14. Tube guide de contact selon la revendication 1, **caractérisé en ce que** la direction d'émission du rayonnement présente une composante directionnelle qui est perpendiculaire à l'axe longitudinal d'une pièce à main.

15. Tube guide de contact selon la revendication 1, **caractérisé en ce que** le déclenchement du rayonnement laser est lié à un autre élément d'actionnement, un interrupteur à pédale notamment, par l'intermédiaire d'une opération logique ET.
